(19) Europäisches Patentamt
European Patent Office
Office européen des brevets

(11) Numéro de publication: **0 507 693 B1**

(12) **FASCICULE DE BREVET EUROPEEN**

(45) Date de publication de fascicule du brevet: **01.03.95**

(51) Int. Cl.6: **A61K 9/113**, A61K 7/00

(21) Numéro de dépôt: **92400934.3**

(22) Date de dépôt: **03.04.92**

# (54) Composition cosmétique sous forme d'émulsion triple.

(30) Priorité: **05.04.91 FR 9104209**

(43) Date de publication de la demande:
**07.10.92 Bulletin 92/41**

(45) Mention de la délivrance du brevet:
**01.03.95 Bulletin 95/09**

(84) Etats contractants désignés:
**AT BE CH DE DK ES FR GB GR IT LI NL PT SE**

(56) Documents cités:
**EP-A- 0 345 075**
**EP-A- 0 422 984**
**WO-A-91/00106**

(73) Titulaire: **L'OREAL**
**14, Rue Royale**
**F-75008 Paris (FR)**

(72) Inventeur: **Vesperini, Laurence**
**62, rue Nationale**
**F-75013 Paris (FR)**
Inventeur: **Fodor, Pierre**
**19, rue des Bures**
**F-92380 Garches (FR)**
Inventeur: **Pouget, Françoise**
**10, rue Commandant Jean Duhail**
**F-92120 Fontenay-sous-Bois (FR)**

(74) Mandataire: **Casalonga, Axel et al**
**BUREAU D.A. CASALONGA - JOSSE**
**Morassistrasse 8**
**D-80469 München (DE)**

EP 0 507 693 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à des compositions cosmétiques se présentant sous forme d'une émulsion triple, à leur procédé de fabrication et à leurs applications dans le domaine de la cosmétologie.

On utilise depuis de nombreuses années des émulsions en cosmétique, notamment pour les produits de traitement cosmétique de la peau. Ces émulsions sont généralement des émulsions huile-dans-eau ou eau-dans-huile (H/E) ou (E/H).

On a déjà décrit dans l'état de la technique des émulsions triples E/H/E ou H/E/H. De telles émulsions présentent cependant de nombreux problèmes tant au niveau de la fabrication qu'au niveau de leur stabilité dans le temps, notamment lorsqu'on introduit dans ces émulsions des substances actives qui ont tendance à déstabiliser ces émulsions.

La demanderesse a découvert, ce qui fait l'objet de l'invention, une émulsion triple présentant des caractéristiques de stabilité et de toucher cosmétique particulièrement remarquables comprenant une phase aqueuse externe continue gélifiée, une phase grasse dispersée dans la phase externe aqueuse et une phase aqueuse interne dispersée dans ladite phase grasse. Cette émulsion triple permet en particulier d'incorporer des matières actives cosmétiques ou dermatologiques dans des quantités supérieures par rapport à ce qu'il était possible d'introduire dans les émulsions de l'état de la technique sans pour autant entraîner une déstabilisation de l'émulsion.

L'invention a donc pour objet une émulsion triple présentant les caractéristiques définies ci-après.

Un autre objet de l'invention est constitué par le procédé de fabrication d'une telle émulsion.

L'invention a également pour objet l'application cosmétique de telles émulsions.

D'autres objets de l'invention apparaîtront à la lecture de la description et des exemples qui suivent.

L'émulsion triple conforme à l'invention est essentiellement caractérisée par le fait qu'elle comporte (A) une phase aqueuse externe continue gélifiée et contenant au moins à titre de gélifiants un polymère ou copolymère d'acide acrylique ou méthacrylique, associé à un polyglycéryl méthacrylate; (B) une phase grasse dispersée dans la phase aqueuse externe et une phase aqueuse (C) dispersée dans la phase grasse.

Conformément à un mode de réalisation préféré de l'invention, la phase aqueuse externe gélifiée représente 40 à 60% en poids de l'émulsion totale, la phase grasse représentant 10 à 25% de l'émulsion totale.

Selon une forme de réalisation préférée de l'invention, on utilise une association de deux gélifiants dans la phase aqueuse externe, constitués d'une part, d'un sel de métal alcalin d'un copolymère acrylique vendu sous la dénomination HOSTACERINE PN 73 par la Société HOECHST et d'autre part, d'un polyglycéryl méthacrylate fabriqué sous la dénomination LUBRAGEL MS par la Société UNITED GUARDIAN INC.

La phase grasse comprend en particulier des huiles végétales, des huiles minérales ou synthétiques. On peut également introduire dans cette phase grasse des cires et/ou des huiles de silicone.

Une forme de réalisation préférée est constituée par l'utilisation dans la phase grasse d'une huile perfluorée en particulier un perfluoropolyéther répondant à la formule :

$$CF_3 — (O - \underset{\displaystyle CF_3}{\underset{|}{C}}F - CF_2)_n — (O - CF_2)_m — OCF_3$$

dans laquelle n et m sont des nombres entiers tels que le poids moléculaire soit compris entre 100 et 10000, le rapport n/m étant compris entre 20 et 40.

Les produits utilisables en particulier dans le cadre de l'invention sont constitués par les produits vendus sous la dénomination FOMBLIN HC, vendus et fabriqués par la Société MONTEFLUOS, Milan, Italie et en particulier les produits FOMBLIN HC/04, FOMBLIN HC/25, FOMBLIN HC/R.

L'émulsion triple conforme à l'invention contient une ou plusieurs substances actives dans des proportions pouvant aller jusqu'à 20% en poids par rapport au poids de l'émulsion totale. Ces substances actives peuvent être constituées notamment par des filtres solaires UV-A, UV-B ou à bande large, par des extraits naturels protéinés tels que les produits à base de collagène, de préférence d'origine marine.

Les émulsions triples contiennent par ailleurs des émulsifiants utilisés pour la réalisation des émulsions eau-dans-huile (E/H) et huile-dans-eau (H/E).

Selon un mode de réalisation préféré du procédé de l'invention, on prépare, dans un premier temps, une émulsion E/H en ajoutant une phase aqueuse à la phase grasse pour l'obtention d'une émulsion E/H, suivie, dans un deuxième temps, par la réalisation de l'émulsion triple par addition de l'émulsion E/H ainsi

obtenue à une seconde phase aqueuse gélifiée qui constitue la phase aqueuse externe de l'émulsion.

L'émulsifiant utilisé pour réaliser l'émulsion E/H comporte de préférence un mélange d'oléate de sorbitan, de lécithine et comme co-émulsifiant un copolymère de dodécyl glycol avec de l'oxyde d'éthylène, tel que plus particulièrement le produit vendu sous la dénomination ELFACOS ST37 par la Société AKZO.

La seconde phase aqueuse gélifiée comporte un émulsifiant choisi de préférence parmi les esters de sucrose.

L'addition des substances actives et des adjuvants habituels tels que parfums, conservateurs, peut être effectuée après réalisation de l'émulsion triple.

Les agents gélifiants utilisés dans la phase aqueuse externe représentent généralement 0,1 à 4,0% en poids par rapport au poids total de l'émulsion triple, et de préférence 0,2 à 2,5% en poids.

Dans les compositions sous forme d'émulsion selon l'invention, on peut prévoir des rapports pondéraux lécithine/oléate compris entre environ 4/2 et 8/2, et de préférence égaux à environ 5/2 et des rapports pondéraux co-émulsifiant copolymère/oléate compris entre 0,2/2 et 1/2, et de préférence égaux à 0,375/2.

Comme déjà indiqué ci-dessus, les compositions sous forme d'émulsions triples conformes à l'invention, présentent des propriétés cosmétiques particulièrement remarquables, notamment au niveau du toucher, ce qui permet de les utiliser comme bases pour appliquer des substances actives cosmétiques sur la peau.

Ces émulsions présentent par ailleurs des caractéristiques de stabilité parfaitement acceptables dans les conditions habituelles de stockage des produits cosmétiques.

Ces émulsions peuvent notamment être utilisées dans des crèmes protectrices, des crèmes dites "barrière", ou bien des compositions pour prévenir des irritations de la peau. Elles peuvent être utilisées dans des produits de traitement cosmétique des matières kératiniques, tels que des crèmes diverses pour produits solaires, fonds de teint, crèmes de jour, etc...

Ces émulsions peuvent également être utilisées dans les applications dermatologiques comme supports pour des agents actifs utilisés en dermatologie.

L'exemple suivant est destiné à illustrer l'invention.

**EXEMPLE**

**PHASE GRASSE (B)**

| | |
|---|---|
| Palmitate d'octyle | 7,00 % |
| Beurre de karité | 1,50 |
| Copolymère séquencé comprenant 22 motifs d'oxyde d'éthylène et 9 motifs de dodécylglycol (ELFACOS ST 37) | 0,20 |
| Diméthicone* (polydiméthylsiloxane) | 3,00 |
| Perfluoropolyméthylisopropyléther (FOMBLIN HC/25) | 1,00 |
| Oléate de sorbitan (SPAN 80 vendu par la Société ICI) | 2,00 |
| Lécithine hydrogénée naturelle | 5,00 |
| Tocophérol | 0,20 |
| Paraméthoxycinnamate de 2-éthylhexyle (PARSOL MCX vendu par la Société GIVAUDAN) | 0,50 |
| Parahydroxybenzoate de propyle | 0,10 |

**PHASE AQUEUSE I (C)**

| | |
|---|---|
| Sorbate de potassium | 0,10 |
| Eau | 20,00 |
| Protéine animale hydrolysée | 10,00 |

**PHASE AQUEUSE II (A)**

| | |
|---|---|
| Copolymère acrylate de sodium/acrylamide (HOSTACERINE PN 73) | 0,25 |

| | |
|---|---|
| Polyglycéryl méthacrylate (LUBRAGEL MS) | 2,00 |
| Méthyl gluceth-20 sesquistéarate* | 1,50 |
| Glycérine | 4,00 |
| Parahydroxybenzoate de méthyle | 0,25 |
| Eau | 36,80 |

* Dénominations CTFA Dictionary.

On prépare l'émulsion triple ci-dessus de la manière suivante :

a) on prépare une émulsion E/H par ajout vers 60 °C de la phase aqueuse C à la phase grasse B sous turbo-agitateur;

b) on ajoute l'émulsion E/H ainsi obtenue à la seconde phase aqueuse gélifiée A.

On ajoute ensuite les substances actives et adjuvants suivants :

| | |
|---|---|
| Collagène | 2.00 |
| Parfum | 0.30 |
| Imidazolidinyl urée | 0.30 |
| Eau | 2.00 |

## Revendications

1. Emulsion triple caractérisée par le fait qu'elle comporte :
   (A) une phase aqueuse externe continue gélifiée et contenant au moins à titre de gélifiants un polymère ou copolymère d'acide acrylique ou méthacrylique, associé à un polyglycéryl méthacrylate;
   (B) une phase grasse dispersée dans la phase aqueuse externe; et
   une phase aqueuse (C) dispersée dans la phase grasse.

2. Emulsion selon la revendication 1, caractérisée par le fait que la phase aqueuse externe gélifiée représente 40 à 60% en poids de l'émulsion totale, la phase grasse représentant 10 à 25% de l'émulsion totale.

3. Emulsion selon la revendication 1 ou 2, caractérisée par le fait que les gélifiants utilisés dans la phase aqueuse externe sont constitués, d'une part, d'un sel de métal alcalin d'un copolymère acrylique et, d'autre part, d'un polyglycéryl méthacrylate.

4. Emulsion selon l'une quelconque des revendications 1 à 3, caractérisée par le fait que la phase grasse comprend des huiles végétales, des huiles minérales ou synthétiques.

5. Emulsion selon la revendication 4, caractérisée par le fait que la phase grasse comporte en outre des cires et/ou des huiles de silicone.

6. Emulsion selon l'une quelconque des revendications 1 à 5, caractérisée par le fait que la phase grasse contient une huile perfluorée.

**7.** Emulsion selon la revendication 6, caractérisée par le fait que l'huile perfluorée est un perfluoropolyéther répondant à la formule :

$$CF_3 — (O-\underset{CF_3}{\underset{|}{C}}F-CF_2)_n — (O-CF_2)_m — OCF_3$$

dans laquelle n et m sont des nombres entiers tels que le poids moléculaire soit compris entre 100 et 10000, le rapport n/m étant compris entre 20 et 40.

**8.** Emulsion selon l'une quelconque des revendications 1 à 7, caractérisée par le fait que la phase grasse contient un co-émulsifiant constitué par un copolymère de dodécylglycol avec l'oxyde d'éthylène.

**9.** Emulsion selon l'une quelconque des revendications 6 à 8, caractérisée par le fait que la phase grasse contient, en combinaison, une huile perfluorée et un co-émulsifiant constitué par un copolymère de dodécylglycol avec l'oxyde d'éthylène.

**10.** Emulsion selon l'une quelconque des revendications 1 à 9, caractérisée par le fait qu'elle comprend 0,1 à 4,0% en poids d'agents gélifiants et de préférence 0,2 à 2,5% en poids, par rapport au poids total de l'émulsion.

**11.** Emulsion selon l'une quelconque des revendications 1 à 10, caractérisée par le fait que la phase grasse contient des émulsifiants constitués par l'oléate de sorbitan et la lécithine, dans des rapports pondéraux lécithine/oléate compris entre 4/2 et 8/2, et de préférence égaux à 5/2.

**12.** Emulsion selon l'une quelconque des revendications 1 à 11, caractérisée par le fait que la phase aqueuse externe gélifiée contient un ester de sucrose a titre d'émulsifiant.

**13.** Emulsion selon l'une quelconque des revendications 1 à 12, caractérisée par le fait qu'elle contient jusqu'à 20% en poids, par rapport au poids total de l'émulsion, de substances actives cosmétologiques ou dermatologiques.

**14.** Procédé de préparation d'une émulsion triple selon l'une quelconque des revendications précédentes, caractérisé par le fait que, dans un premier temps, on réalise une émulsion eau-dans-huile en ajoutant une phase aqueuse à une phase grasse pour obtenir une émulsion eau-dans-huile et qu'on réalise ensuite, dans un deuxième temps, l'émulsion triple par addition de l'émulsion eau-dans-huile ainsi obtenue à une seconde phase aqueuse gélifiée contenant à titre de gélifiants un polymère ou copolymère d'acide acrylique ou méthacrylique associé à un polyglycéryl méthacrylate.

**15.** Procédé selon la revendication 14, caractérisé par le fait qu'on introduit dans la phase grasse une huile perfluorée.

**16.** Procédé selon la revendication 14, caractérisé par le fait qu'on introduit dans la phase grasse un co-émulsifiant constitué par un copolymère de dodécylglycol et d'oxyde d'éthylène.

**17.** Procédé selon l'une quelconque des revendications 14 à 16, caractérisé par le fait qu'on introduit dans la phase grasse, en combinaison, une huile perfluorée et un co-émulsifiant constitué par un copolymère de dodécylglycol et d'oxyde d'éthylène.

**18.** Procédé selon l'une quelconque des revendications 14 à 17, caractérisé par le fait que l'on utilise comme émulsifiants pour réaliser l'émulsion eau-dans-huile, un mélange d'oléate de sorbitan et de lécithine.

**19.** Procédé selon l'une quelconque des revendications 14 à 18, caractérisé par le fait qu'on ajoute, dans la phase aqueuse gélifiée, à titre d'émulsifiant, un ester de sucrose.

**20.** Procédé selon l'une quelconque des revendications 14 à 19, caractérisé par le fait que l'on introduit au moins une substance active après réalisation de l'émulsion triple.

**21.** Composition cosmétique destinée à être appliquée sur la peau, caractérisée par le fait qu'elle comporte au moins une émulsion triple telle que définie dans l'une quelconque des revendications 1 à 13.

**22.** Composition destinée à être utilisée en dermatologie et contenant des agents dermatologiquement actifs, caractérisée par le fait que son support est constitué par une émulsion triple telle que définie dans l'une quelconque des revendications 1 à 13.

## Claims

**1.** Triple emulsion characterised in that it comprises:
(A) a continuous gelled outer aqueous phase, containing, as gelling agent, at least one acrylic or methacrylic acid polymer or copolymer, combined with a polyglyceryl methacrylate;
(B) a fatty phase dispersed in the outer aqueous phase;
and an aqueous phase (C) dispersed in the fatty phase.

**2.** Emulsion according to Claim 1, characterised in that the gelled outer aqueous phase represents 40 to 60% by weight of the total emulsion, the fatty phase representing 10 to 25% of the total emulsion.

**3.** Emulsion according to Claim 1 or 2, characterised in that the gelling agents used in the outer aqueous phase consist, on the one hand, of an alkali metal salt of an acrylic copolymer and, on the other hand, of a polyglyceryl methacrylate.

**4.** Emulsion according to any one of Claims 1 to 3, characterised in that the fatty phase comprises vegetable oils, mineral or synthetic oils.

**5.** Emulsion according to Claim 4, characterised in that the fatty phase comprises in addition, waxes and/or silicone oils.

**6.** Emulsion according to any one of Claims 1 to 5, characterised in that the fatty phase contains a perfluorinated oil.

**7.** Emulsion according to Claim 6, characterised in that the perfluorinated oil is a perfluoropolyether of the formula:

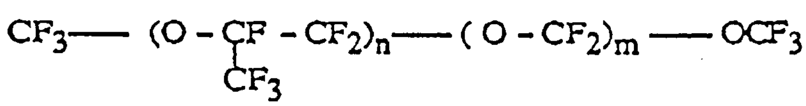

in which n and m are integers such that the molecular weight is between 100 and 10,000, the n/m ratio being between 20 and 40.

**8.** Emulsion according to any one of Claims 1 to 7, characterised in that the fatty phase contains a co-emulsifier consisting of a dodecyl glycol and ethylene oxide copolymer.

**9.** Emulsion according to any one of Claims 6 to 8, characterised in that the fatty phase contains, in combination, a perfluorinated oil and a co-emulsifier consisting of a dodecyl glycol and ethylene oxide copolymer.

**10.** Emulsion according to any one of Claims 1 to 9, characterised in that it comprises 0.1 to 4.0% by weight of gelling agents and preferably 0.2 to 2.5% by weight, relative to the total weight of the emulsion.

**11.** Emulsion according to any one of Claims 1 to 10, characterised in that the fatty phase contains emulsifiers consisting of sorbitan oleate and lecithin, in lecithin/oleate weight ratios of between 4/2 and

8/2, preferably equal to 5/2.

**12.** Emulsion according to any one of Claims 1 to 11, characterised in that the gelled outer aqueous phase contains a sucrose ester as emulsifier.

**13.** Emulsion according to any one of Claims 1 to 12, characterised in that it contains up to 20% by weight, relative to the total weight of the emulsion, of cosmetic or dermatological active substances.

**14.** Process for preparing a triple emulsion according to any one of the preceding claims, characterised in that in a first stage, a water-in-oil emulsion is prepared by adding an aqueous phase to a fatty phase so as to obtain a water-in-oil emulsion and in that the triple emulsion is then prepared, in a second stage, by adding the water-in-oil emulsion thus obtained to a second gelled aqueous phase containing, as gelling agent, an acrylic or methacrylic acid polymer or copolymer combined with a polyglyceryl methacrylate.

**15.** Process according to Claim 14, characterised in that a perfluorinated oil is introduced into the fatty phase.

**16.** Process according to Claim 14, characterised in that a co-emulsifier consisting of a dodecyl glycol and ethylene oxide copolymer is introduced into the fatty phase.

**17.** Process according to any one of Claims 14 to 16, characterised in that a perfluorinated oil and a co-emulsifier consisting of a dodecyl glycol and ethylene oxide copolymer are introduced, in combination, into the fatty phase.

**18.** Process according to any one of Claims 14 to 17, characterised in that a sorbitan oleate and lecithin mixture is used as emulsifier to prepare the water-in-oil emulsion.

**19.** Process according to any one of Claims 14 to 18, characterised in that a sucrose ester is added, as emulsifier, to the gelled aqueous phase.

**20.** Process according to any one of Claims 14 to 19, characterised in that at least one active substance is introduced after preparation of the triple emulsion.

**21.** Cosmetic composition intended to be applied to the skin, characterised in that it comprises at least one triple emulsion as defined in any one of Claims 1 to 13.

**22.** Composition intended to be used in dermatology, and containing dermatologically active agents, characterised in that its carrier consists of a triple emulsion as defined in any one of Claims 1 to 13.

**Patentansprüche**

**1.** Dreier-Emulsion,
dadurch **gekennzeichnet**, daß
sie umfaßt:
(A) eine äußere wässrige Phase, die dauerhaft geliert ist und mindestens als Geliermittel ein Polymer oder Copolymer aus Acryl- oder Methacrylsäure enthält, das an ein Polyglycerylmethacrylat assoziiert ist;
(B) eine Fettphase, die in der äußeren wässrigen Phase dispergiert ist; sowie
(C) eine wässrige Phase, die in der Fettphase dispergiert ist.

**2.** Emulsion gemäß Anspruch 1,
dadurch **gekennzeichnet**, daß
die gelierte äußere wässrige Phase 40 bis 60 Gew.% der gesamten Emulsion darstellt, wobei die Fettphase 10 bis 25% der gesamten Emulsion ausmacht.

**3.** Emulsion gemäß Anspruch 1 oder 2,
dadurch **gekennzeichnet**, daß

die in der äußeren wässrigen Phase verwendeten Geliermittel einerseits aus einem Alkalimetallsalz eines Acrylcopolymers und andererseits aus einem Polyglycerylmethacrylat zusammengesetzt sind.

**4.** Emulsion gemäß jedem der Ansprüche 1 bis 3,
dadurch **gekennzeichnet**, daß
die Fettphase pflanzliche, mineralische oder synthetische Öle umfaßt.

**5.** Emulsion gemäß Anspruch 4,
dadurch **gekennzeichnet**, daß
die Fettphase ausserdem Wachse und/oder Öle von Silikon enthält.

**6.** Emulsion gemäß jedem der Ansprüche 1 bis 5,
dadurch **gekennzeichnet**, daß
die Fettphase ein perfluoriertes Öl enthält.

**7.** Emulsion gemäß Anspruch 6,
dadurch **gekennzeichnet**, daß
das perfluorierte Öl ein Perfluorether der Formel ist:

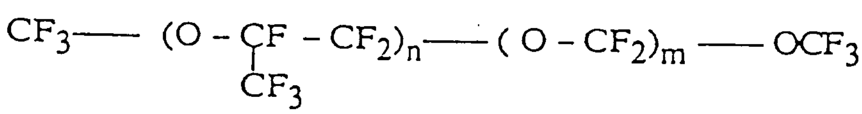

worin n und m ganze Zahlen einer solchen Größe sind, daß das Molekulargewicht 100 bis 10000 beträgt, wobei das Verhältnis n/m 20 bis 40 beträgt.

**8.** Emulsion gemäß jedem der Ansprüche 1 bis 7,
dadurch **gekennzeichnet**, daß
die Fettphase einen Coemulgator aus einem Copolymer von Dodecylglycol mit Ethylenoxid enthält.

**9.** Emulsion gemäß jedem der Ansprüche 6 bis 8,
dadurch **gekennzeichnet**, daß
die Fettphase, in Kombination, ein perfluoriertes Öl und einen Coemulgator aus einem Copolymer von Dodecylglycol mit Ethylenoxid enthält.

**10.** Emulsion gemäß jedem der Ansprüche 1 bis 9,
dadurch **gekennzeichnet**, daß
sie 0,1 bis 4,0, vorzugsweise 0,2 bis 2,5, Gewichtsprozent Geliermittel enthält, bezogen auf das Gesamtgewicht der Emulsion.

**11.** Emulsion gemäß jedem der Ansprüche 1 bis 10,
dadurch **gekennzeichnet**, daß
die Fettphase Emulgatoren aus Sorbitanoleat und Lecithin in Mengenverhältnissen von Lecithin/Oleat von 4/2 bis 8/2, vorzugsweise von 5/2, enthält.

**12.** Emulsion gemäß jedem Anspruch 1 bis 11,
dadurch **gekennzeichnet**, daß
die gelierte äußere wässrige Phase einen Succroseester als Emulgator enthält.

**13.** Emulsion gemäß jedem Anspruch 1 bis 12,
dadurch **gekennzeichnet**, daß
sie bis zu 20 Gew.%, bezogen auf das Gesamtgewicht der Emulsion, kosmetologische oder dermatologische Wirksubstanzen enthält.

**14.** Verfahren zur Herstellung einer Dreier-Emulsion gemäß jedem der vorhergehenden Ansprüche,
dadurch **gekennzeichnet**, daß

man, in einer ersten Stufe, eine Wasser-in-Öl-Emulsion herstellt, indem man zum Erhalt der Wasser-in-Öl-Emulsion eine wässrige Phase zu einer Fettphase gibt, und man sodann, in einer zweiten Stufe, die Dreier-Emulsion durch Zugabe der so erhaltenen Wasser-in-Öl-Emulsion zu einer gelierten wässrigen zweiten Phase herstellt, die als Geliermittel ein Polymer oder Copolymer von Acryl- oder Methacrylsäure enthält, welche an ein Polyglycerylmethacrylat assoziiert sind.

**15.** Verfahren gemäß Anspruch 14,
dadurch **gekennzeichnet**, daß
man in die Fettphase ein perfluoriertes Öl einbringt.

**16.** Verfahren gemäß Anspruch 14,
dadurch **gekennzeichnet**, daß
man in die Fettphase einen Coemulgator aus einem Copolymer von Dodecylglycol und Ethylenoxid einbringt.

**17.** Verfahren gemäß jedem Anspruch 14 bis 16,
dadurch **gekennzeichnet,** daß
man in die Fettphase, in Kombination, ein perfluoriertes Öl und einen Coemulgator aus einem Copolymer aus Dodecylglycol und Ethylenoxid einbringt.

**18.** Verfahren gemäß jedem Anspruch 14 bis 17,
dadurch **gekennzeichnet**, daß
man als Emulgatoren zur Herstellung der Wasser-in-Öl-Emulsion eine Mischung aus Sorbitanoleat und Lecithin verwendet.

**19.** Verfahren gemäß jedem Anspruch 14 bis 18,
dadurch **gekennzeichnet,** daß
man der gelierten wässrigen Phase als Emulgator einen Succroseester zufügt.

**20.** Verfahren gemäß jedem Anspruch 14 bis 19,
dadurch **gekennzeichnet,** daß
man mindestens eine Wirksubstanz einbringt, nachdem die Dreier-Emulsion hergestellt worden ist.

**21.** Kosmetische Zusammensetzung zur Aufbringung auf die Haut,
dadurch **gekennzeichnet,** daß
sie mindestens eine in jedem der Ansprüche 1 bis 13 definierte Dreier-Emulsion umfaßt.

**22.** Zusammensetzung zur Verwendung in der Dermatologie, welche dermatologisch wirksame Mittel enthält,
dadurch **gekennzeichnet,** daß
das Trägermittel aus einer in jedem der Ansprüche 1 bis 13 definierten Dreier-Emulsion zusammengesetzt ist.